# EUROPEAN PATENT SPECIFICATION

(11) **EP 1 216 237 B1**
(45) Date of publication and mention of the grant of the patent: **17.12.2003**
(21) Application number: 00966338.6
(22) Date of filing: 22.09.2000
(51) Int. Cl.: C07D 307/88

(54) **PROCESS FOR THE PREPARATION OF MYCOPHENOLIC ACID AND DERIVATIVES THEREOF**
VERFAHREN ZUR HERSTELLUNG VON MYCOPHENOLSÄURE UND DERIVATEN DAVON
ELABORATION D'ACIDE MYCOPHENOLIQUE ET DE DERIVES DE CET ACIDE

(30) Priority: 23.09.1999 HU 9903226
(43) Date of publication of application: 26.06.2002
(73) Proprietor: IVAX Drug Research Institute Ltd., 1045 Budapest (HU)
(72) Inventor: BARTA, István, H-1142 Budapest (HU); BOROS, Sándor, H-2134 Sz d (HU); AMBRUS, Gábor, H-1025 Budapest (HU); HORV TH, Gyula, H-1052 Budapest (HU); SZAB , Antal, H-2000 Szentendre (HU); SZAB , István, Mihály, H-1111 Budapest (HU); JEKKEL, Antónia, H-1071 Budapest (HU); K NYA, Attila, H-5000 Szolnok (HU); M ZES, Julianna, H-1173 Budapest (HU); SAL T, János, H-1151 Budapest (HU); SOMOGYI, György, H-1148 Budapest (HU)
(74) Representative: Hay, Martin Alexander
(86) International application number: HU0000099
(87) International publication number: WO01021607

(56) References cited:
- JONES, DERRICK FLEET ET AL: "Preparation and antitumor properties of analogs and derivatives of mycophenolic acid" J. MED. CHEM. (1971), 14(4), 305-11 , XP002165088
- SUZUKI, SEIKICHI ET AL: "Antitumor activity of derivatives of mycophenolic acid" J. ANTIBIOT. (1976), 29(3), 275-85 , XP000067668
- SWEENEY, MARTIN J. ET AL: "Experimental antitumor activity and preclinical toxicology of mycophenolic acid" CANCER RES. (1972), 32(9), 1795-802 , XP002165090 cited in the application
- MCCORKINDALE, N. J. ET AL: "4-Methyl- and 7-methylphthalan-1-one derivatives of mycophenolic acid. Use in establishing the distribution of acetate derived hydrogens" TETRAHEDRON (1981), 37(11), 2131-6 , XP002165091
- JONES, DERRICK FLEET ET AL: "Microbial modification of mycophenolic acid" J. CHEM. SOC., C (1970), (12), 1725-37 , XP002165092 cited in the application

## Description

This invention relates to a microbiological process for the preparation of mycophenolic acid of the formula (I) 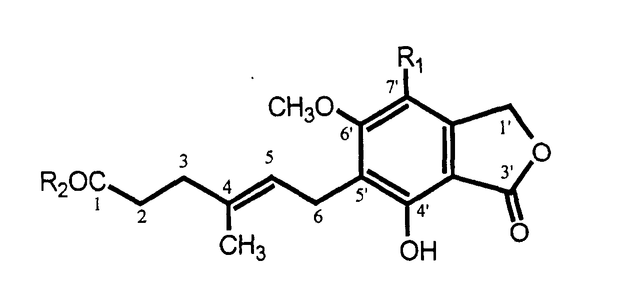 (wherein R₁ means methyl group and R₂ stands for hydroxyl group) and derivatives thereof [in the formula (I) R₁ means methyl or hydroxymethyl group and R₂ is an amino group]. Mycophenolic acid of the formula (I) [(E)-6-(1,3-dihydro-4-hydroxy-6-methoxy-7-- methyl-3-oxo-5-isobenzofuranyl)-4-methyl-4-hexenoic acid] is therapeutically used due to its immunosuppressive effect.

Mycophenolic acid had been first reported in 1896 in the fermentation broth of *Penicillium glaucum* [E. L. Jones et al.: J. Invest. Dermatol. **65**, 537 (1975)] and later it was isolated also from the fermentation broths of other species of the *Penicillium* genus, e.g. *P. brevicompactum, P. stoloniferum, P. echinulatum, P. roqueforti* and *P. viridicatum*. The structure of mycophenolic acid was determined by J. M. Birkinshaw et al. [Biochem. J. **50**, 630 (1952)] in 1952. After the discovery of mycophenolic acid its antibacterial activity was also described, but pathogenic *Staphylococcus* strains rapidly became resistant to it; therefore, it was not further developed in this field [E. P. Abraham et al.: Biochem. J. **39**, 398 (1945)]. The antifungal activity of the compound against some *Epidermophyton* and *Trichophyton* strains was also shown. Similarly, its antiviral effect was also proven *inter alia* on *the Herpes simplex* virus [R. H. Williams et al.; J. Antibiot. **21**, 463 (1968)]. Likewise an antitumour activity of it was published, which was begun to be intensively studied in the recent years [Y. Sidi et al.; Br. J. Cancer 58, 61 (1988)]. Its morpholinoethyl ester derivative described in the second half of the eighties has been approved for therapeutical use as an immunosuppressive agent.

The wide-range biological efficiency of mycophenolic acid can be explained by its inhibitory effect against the activity of the inosine-5'-monophosphate dehydrogenase and guanine-5'-mono-phosphate synthetase enzymes, which results in a decrease of the guanine synthesis. In some cells the guanine synthesis can be accomplished also in an other pathway by the hypoxanthine guanine--phosphoribosyl transferase enzyme. This enzyme is absent, however, from the T and B lymphocytes and, therefore, their growth is selectively inhibited by mycophenolic acid.

At the beginning of the 1970s a number of mycophenolic acid derivatives have been prepared. Modifications were made both on the isobenzofuranyl ring as well as the 4-methyl-4-hexenoic acid side chain by microbiological as well as chemical means [D. F. Jones et al.: J. Chem. Soc. 1725 (1970)].

Jones and Mills [J. Med. Chem. 14(4): 305, (1971)], and Suzuki, *et al,* [J. Antibiot. 29(3):275, (1976)] describe the preparation and antitumor activity of mycophenolic acid and derivatives of mycophenolic acid. Both reports purport that the greatest antitumor activity observed resided with the parent compound, mycophenolic acid, and that none of the substitutions derived showed any enhanced tumoricidal activity.

Some mycophenolic acid derivatives have shown an activity approximating that of mycophenolic acid [M. J. Sweeney et al.: Cancer Research **32**, 1795 (1972)] in antitumour tests. There were mycophenolic acid derivatives which exerted a higher inhibitory effect on inosine-5'-monophosphate dehydrogenase than mycophenolic acid [M. J. Sweeney et al.: Cancer Research 32, 1803 (1972)].

In one aspect the invention aimed at the selection and genetic improvement of a microorganism, by the use of which mycophenolic acid can be produced more advantageously in comparison to the processes known in the literature. From an other aspect, the invention aimed at a microbiological process of preparing mycophenolic acid and novel, potentially therapeutically effective mycophenolic acid derivatives.

In the course of our investigations for the search of new active agents of microbiological origin, microorganisms isolated from soil samples collected at various geographical sites were investigated. Within the framework of the antifungal screening program a fungal strain was isolated, the fermentation broth of which contained mycophenolic acid. A mutant strain producing high concentration of mycophenolic acid was prepared from this strain by using mutation-selection methods. Ultraviolet irradiation and N-methyl-N-- nitro-N'-nitrosoguanidine were applied as mutagenic agents.

The biosynthesis of mycophenolic acid has been described in the cases of *Penicillium glaucum, Penicillium stoloniferum, Penicillium brevicompactum, Penicillium scabrum, Penicillium nagemi, Penicillium patrismei, Penicillium griseobrunneum* and *Penicillium viridicatum* strains. In the course of the biosynthesis of mycophenolic acid the isobenzofuran moiety of the molecule is formed in a way different from the side chain attached thereto. The biosynthesis of the side chain follows the synthesis of the steroid skeleton up to farnesyl pyrophosphate. The isobenzofuran moiety is formed from one acetyl-CoA and three malonyl-CoA-s. The methyl group on the aromatic ring is incorporated from S-adenosylmethionine into the molecule. Farnesyl pyrophosphate, from which the side chain of mycophenolic acid is formed after splitting off a 8-- membered chain, is attached to C-6 of the isobenzofuran ring. The last step of the biosynthesis comprises the formation of a methoxyl group from S-adenosylmethionine [W. L. Muth et al.: Antimicrobial Agents and Chemotherapy **8**, 321 (1975)].

All mycophenolic acid producing strains described in the literature belong to the *Asymmetrica* section within the *Penicillium* genus. Based on the taxonomical determination the strain isolated by us belongs to the *Monoverticillata* section within the *Penicillium* genus and can be classified to the Penicillium *waksmani* species, the mycophenolic acid-producing capability of which has not yet been published in the literature.

The taxonomical determination is given in the following.

### General cultural-morphological characteristics:

The novel strain isolate is a characteristic representative of *Penicillium* genus in the aspect of building up of its conidiophores and the arrangement of branches, metulas as well as fialides thereof. Its sporuling air mycelium is usually velvet-like, well developed. In a mature state it is dark brownish-grey in a starch + ammonium sulphate agar; light brownish-grey on Sabouraud's peptone + glucose agar; dark grey on Sabouraud's glucose agar; light green on asparagine + glycerol; brownish black on Czapek's (nitrate + sucrose) agar with cattle blood; dark brownish grey on the simple Czapek's agar; darkening green on the malt agar; darkening brownish grey on the malt extract + yeast extract + glucose agar; dark brownish grey on oat flake agar. In the beginning, a large number of hyphes is frequently green or bluish green on culture media, where the mature air mycelium is grey or brownish grey. In general the substrate mycelium of the strain is colourless or pale yellowish-brown. Production of a soluble pigment could not be observed on any of the media. The surface of the colonies is less wrinkling. The average thickness of the air mycelium does not exceed 1 to 3 mm. The production of melanoid pigment (tyrosinase activity) is negative.

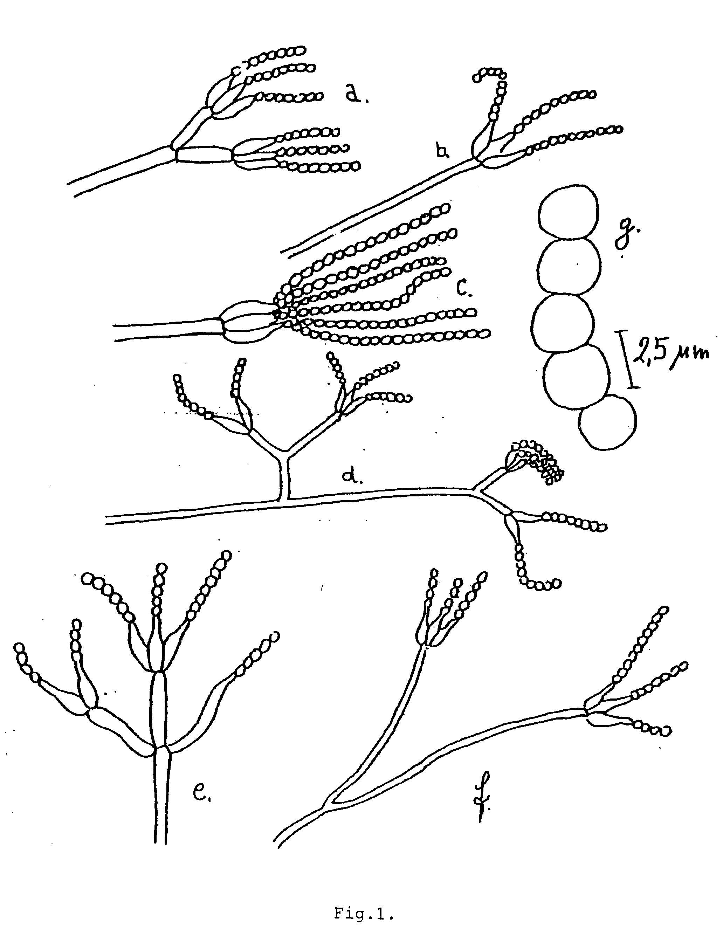

The length of conidiophores is extremely various: they frequently grow out directly from the substrate mycelium, another time they are side embranchments of a longer axial filament (see Figure 1: conidiophore types of a to f; a, d, e and f on asparagine + glycerol agar; b on malt agar; while c on starch + ammonium sulphate agar; g is a conidium chain). The strain can be classified into the group of *Monoverticillata* species, considering that in overwhelming majority it bears brushes consisting of a single fialide whorl. Brushes of this *Monoverticillata* type are very frequently arranged in a "divaricata" manner (see Figure 1). Conidiophores of *Biverticillium* type can also be observed in each case separately (see Figure 1). The whorls construed by fialides can be 2 to 8 (in the most cases 3) membered. The conidia are round, in the most cases they have a smooth surface with a size of 2 to 25 µm. The number of individual conidia in the conidium chains may surpass 20.

### Physiological properties:

Aesculin hydrolysis: positive. Production of hydrogen sulfide: negative. NaCl tolerance: up to 3 % as a maximum. pH-tolerance: from 3.0 to 9.0. The formation of nitrite from nitrate, evolving of ammonia or gas could not be detected. Catalase test: positive. Oxidase test: positive. Urea decomposition: positive. Lecithinase and gelatinase activity: positive. Starch hydrolysis is weak. Tween-20 hydrolysis: positive, but the hydrolysis of Tween-40 and Tween-60 is very weak. From the sodium salts of organic acids it grows advantageously on benzoate, salicylate, weakly on acetate, tartarate and succinate. It does not grow at all or only in traces on malonate, pyruvate, lactate and citrate. It excellently utilizes glucose, fructose, arabinose, xylose, rhamnose, sucrose, raffinose, mannitol and inositol. A strong acid production was observed (by using bromo-cresolpurple indicator on the culture media of R. E. Gordon): on glucose, sucrose, rhamnose, dextrin, melibiose, maltose, raffinose, fructose, inositol, inulin, glycerol, xylose, dulcitol and mannitol. The acid production was weak on galactose, arabinose and salicin.

### Taxonomical position:

The mycophenolic acid-producing *Penicillium* strain isolated by us can be considered to be a near variant of *Penicillium waksmani* species. This identification is also verified by the data of Table 1 below.

**Table 1**

| A comparison of the standard description of *Penicillium waksmani* with the diagnostical characteristics of the mycophenolic acid-- producing *Penicillium* strain isolated by us | | |
|---|---|---|
| | *Penicillium waksmani* | Novel *Penicillium* isolate |
| Colour of mature aerial mycelium | Bluish green→dark green→oily grey→grey | Bluish green→dark green→grey→ brownish grey |
| Substrate mycelium | colourless | colourless |
| Soluble pigments | none | none |
| Form and size of conidia | round; 2 to 2.5 µm | round; 2 to 2.5 µm |
| Brushes consisting of a single fialide whorl | prevailing type | prevailing type |
| Conidiophores of Biverticillium type | occur | occur |

In one aspect the invention is based on the recognition that the mycophenolic acid-producing *Penicillium waksmani* strain isolated by us, which was deposited under No. NCAIM (P)F 001269 at the National Collection of Agricultural and Industrial Microorganisms produces high concentrations of mycophenolic acid under suitable fermentation conditions after developing it by mutation-selection methods.

Further, the invention is based on the recognition that the cultures of strains of *Streptomyces griseoruber* No. 1/6 (deposited as *Streptomyces sp.* No. 1/6) and *Streptomyces resistomycificus* No. 1/28, respectively, isolated similarly by us from soil samples, are also capable to transform the mycophenolic acid, formed to a compound of general formula (I), wherein R₁ means methyl or hydroxymethyl group and R₂ stands for amino group.

### Taxonomic description of the Streptomyces strain No. 1/6

*Cultural-morphological diagnostic properties*: On oatmeal agar a well developed aerial mycelium of typical red colourization (Red-colour series; "Cinnamomeus"-colour-series) covers the substrate mycelial surface. The colour of the latter is yellowish. Soluble pigments are not visible. Observing the red spore-mass, the occurrence of many Retinaculum apertum-type sporophores can be detected. On Czapek-agar: The colonies are characterized by strongly developed reddish aerial mycelium, reddish-yellow substrate mycelium and faint reddish diffusible pigments. On potato slices the brown substrate mycelium is covered by reddish aerial spore-mass, while the colour of the potato became dark brownish red. On soy-peptone agar under the well developed reddish aerial mycelium, the substrate mycelium as well as the medium itself dark brown or black. On malate agar plates the yellowish aerial mycelium remained mostly sterile and under the brownish substrate mycelium the diffusion of an intense reddish brown soluble pigment may be observed. On yeast-iron agar strain No.1/6 shows extremely positive melanoid pigment production. Similarly, it proved to be chromogenic also on tyrosine agar but with weaker intensity.

*Physiological properties*: Strain No. 1/6 does not decompose cellulose. Positive utilization of glucose, fructose, sucrose, rhamnose and i-inositol, furthermore relatively weak growth but still positive utilization with xylose and arabinose, as sole sources in the medium, have been observed. Very weak growth (negative utilization) with raffinose was detected.

*Taxonomic position:* Strain No. 1/6 as a typical red *Streptomyces* (spore mass colour in the Red-series, colour of the substrate mycelium: yellow-brown + red, reddish soluble pigment) characterized with Retinaculum apertum (spirale) sporophores shows a considerable cultural-morphological similarity to the species *Streptomyces griseoruber* Yamaguchi and Saburi (1955) which belongs to the Cluster 18 (23) of the numerical classification system of Kampfer et al. [J. Gen. Microbiol. **137**, 1831-1891 (1991)]. Regarding some physiological differences, however, further studies will be necessary to clarify its closer relationships.

### Taxonomic description of the Streptomyces strain No. 1/28

Cultural- *as well as light- and electronmicroscopic morphological diagnostic properties*: On oatmeal-agar plates the aerial mycelium is weakly developed, its colour at first white later light grayish and can not be accurately characterized according to the colour-series categories of the International Streptomyces Project [(Shirling and Gottlieb, Int. J. Syst. Bact., **16**, 313-340 (1966)]. According to the results of microscopic observations the hyphae proved to be sterile, their differentiation into sporophores and conidia were not observed. Medium development of the substrate mycelium and production of light yellowish-brown soluble pigments were detected. On yeast extract- malt extract agar weakly developed white and sterile aerial mycelium, reddish-brown substrate mycelium and light yellowish diffusible pigments were observed. On inorganic salts - starch agar plates the well developed dark reddish-gray substrate mycelium was covered with relatively more developed aerial mycelium the colour of which changed from white into a yellowish-gray mass of short (Retinaculum apertum-type) spiral sporophores and spores of smooth surfaces. The presence of soluble pigments were not detected. The cultures of this strain (1/28) produced both on peptone-yeast-iron agar and tyrosine agar dark brown or black melanoid pigments.

*Physiological properties:* Good growth was observed on cellulose, but without the visible degradation of the cellulose fibres. In nitrate broth it showed good growth, but the presence of nitrites, ammonia or development of gas were not detected. It proved to be able to tolerate NaCl concentrations to 6 %, and pH values between 5 and 11. Positive aesculine hydrolysis- and urease-tests were detected. Good growth was observed with glucose, fructose, arabinose, xylose, rhamnose, sucrose, mannitol, raffinose and mesoinositol as sole sources of carbon in the medium. Weak growth was observed with Na-citrate, Na-malonate, Na-pyruvate, Ca-lactate and Ca-succinate. The growth was only in traces with Na-acetate, Na-tartrate and Na-benzoate.

*Taxonomic position*: Although the correct identification of the colour of aerial mycelium was not possible, on the basis of the most important diagnostic properties we can designate this strain as a weakly sporulating variant of the species *Streptomyces resis tomycificus* Lindenbein, 1952 (Str. *violaceus* according to Kampfer et al., 1991).

Thus, the invention refers to a microbiological process for the preparation of compounds of the general formula (I), 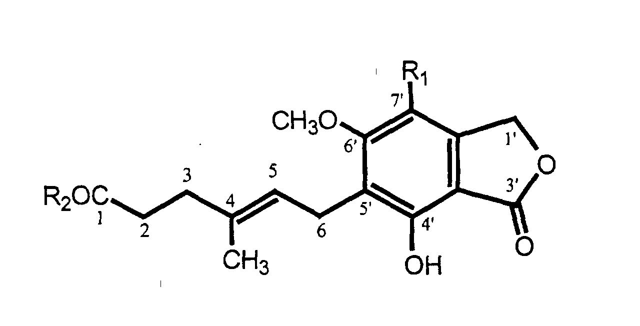 wherein R₁ means methyl or hydroxymethyl group and R₂ stands for hydroxyl or amino group, which comprises growing a *Penicillium waksmani* fungal strain able to biosynthesising mycophenolic acid at 22°C to 30°C on a culture medium containing assimilable carbon and nitrogen sources as well as mineral salts, separating the compound of general formula (I), wherein R₁ means methyl group and R₂ stands for hydroxyl group, from the fermentation broth and, if desired, purifying it and, if desired, fermenting it
a) by using the cultures of the *Streptomyces griseoruber* No. *1*/*6* actinomycete strain deposited at the National Collection of Agricultural and Industrial Microorganisms as Streptomyces *sp.* No. 1/6 under No. NCAIM (P)B 001275 to prepare a compound of general formula (I), wherein R₁ means methyl group and R₂ stands for amino group; or
b) by using the cultures of *Streptomyces resistomycificus* actinomycete strain No. 1/28 deposited at the National Collection of Agricultural and Industrial Microorganisms under No. NCAIM (P)B 001276 to prepare a compound of general formula (I), wherein R₁ means hydroxymethyl group and R₂ stands for amino group
on a culture medium containing assimilable carbon and nitrogen sources as well as mineral salts under sunk, aerated conditions up to completion of the bioconversion, then separating the compound of general formula (I) formed from the culture and, if desired, purifying it.

According to the invention, the cultures of *Penicillium waksmani* strain advantageously utilizing glucose, maltose, sucrose, glycerol, malt extract and water-soluble starch as carbon sources is used for the production of mycophenolic acid. Yeast extract, peptone, meat extract, casein, trypcasin, soybean flour, corn steep liquor, sodium nitrate or ammonium sulfate can be used as nitrogen sources.

In addition to the above carbon and nitrogen sources mineral salts, e.g., magnesium sulfate, amino acids, vitamins and antifoam agents, may also be present in the culture media used for producing mycophenolic acid.

According to a preferred embodiment of the process of invention for the preparation of mycophenolic acid, a spore suspension is prepared with distilled water from the slant agar cultures of *Penicillium waksmani* strain deposited at the National Collection of Agricultural and Industrial Microorganisms under No. NCAIM (P)F 001269 or a mutant strain thereof, which is able to biosynthesising mycophenolic acid. After inoculating 5 ml of this spore suspension into a seed culture medium, a productive culture medium is inoculated with the 3-day inoculum culture grown at 24°C to 28°C, preferably at 25°C, then incubated at 22°C to 28°C, preferably at 25°C, for 7 days. The cultivation was carried out under aerobic conditions, during which the pH value changed between 3.5 and 7.5. In the main period of fermentation the air inlet was 120 1/hour, the rate of stirring was 400 revolutions/minute.

During the fermentation the active substance content of the fermentation broth was determined by using high pressure liquid chromatography (HPLC) or densitometry after thin layer chromatography (TLC). When the culture contained the highest concentration of mycophenolic acid, the fermentation was stopped. For the analyses by high pressure liquid chromatography, the centrifuged supernatants of fermentation broth samples diluted to the 4-fold with methanol were applied [equipment: LKB isocratic system; column: Nucleosil C₁₈ 5 µm (BST); intermediate column: 40x4 mm; analytical column: 250x4.6 mm; thermostated at 25 °C; measurement at 238 nm; eluent: acetonitrile - distilled water adjusted to pH 2 with phosphoric acid (60:40); flow rate: 1.0 ml/min; injection volume: 10 µl]. The retention time of mycophenolic acid is 15.8 minutes in this system.

According to a preferred embodiment of the process of invention for the preparation of a compound of general formula (I), wherein R₁ means methyl group and R₂ stands for amino group, a spore suspension is prepared with distilled water from a slant agar culture of the *Streptomyces griseoruber* strain No. 1/6. A seed culture medium was inoculated with this spore suspension, then a productive culture medium was inoculated with the 3-day inoculum culture grown at 25°C to 37°C, preferably at 28°C, which was then incubated at 25°C to 32°C, preferably at 28°C, for 3 days. After adding mycophenolic acid to the culture, the cultivation was continued for additional 7 days.

According to another preferred embodiment of the process of invention for the preparation of a compound of general formula (I), wherein R₁ means hydroxymethyl group and R₂ stands for amino group, a spore suspension is prepared with distilled water from the slant agar culture of the *Streptomyces resistomycificus* strain No. 1/28. A seed culture medium was inoculated with this spore suspension, then a productive culture medium was inoculated with the 3-day inoculum culture grown at 25°C to 37°C, preferably at 28°C, for 3 days. After adding mycophenolic acid to the culture, the cultivation was continued for additional 7 days.

The processes of the invention are illustrated by the following examples.

### Example 1

The Penicillium *waksmani* mutant strain [NCAIM (P)F 001269] biosynthesising mycophenolic acid in a high concentration was prepared in a mutation-selection experiment carried out by means of N-methyl-N-nitro-N'-nitrosoguanidine. The mycophenolic acid biosynthesising Penicillium *waksmani* strain isolated from a soil sample was grown on MS agar slant at 25°C for 10 days.

| Composition of the culture medium MS: | |
|---|---|
| glucose | 4 g |
| malt extract | 10 g |
| yeast extract | 4 g |
| agar | 20 g |
| in 1000 ml of distilled water | |

The spores were washed off from the agar slant culture with 5 ml of a sterile 0.1 M phosphate buffer (pH 8.0). To the spore suspension N-methyl-N-nitro-N'-nitrosoguanidine was added in a final concentration of 1 mg/ml. The suspension was shaken at 28°C at 150 rpm for 35 minutes. Subsequently, the spores were sedimented by centrifuging at a rate of 5000 rpm and then resuspended in sterile distilled water. The suspension was spred onto MS agar plates. After incubating the plates at 25 °C for 10 days the grown colonies were inoculated onto MS culture medium. The mycophenolic acid-- producing capability of the grown agar slant cultures was screened in the manner described in Example 2, in shaken flask experiments.

### Example 2

The spores from a 7- to 10-days culture of NCAIM (P)F 001269 *Penicillium waksmani* strain grown on slant agar containing malt extract and yeast extract were washed off with 5 ml of sterile distilled water, then 100 ml of MI inoculum culture medium sterilized in a 500 ml Erlenmeyer flask were inoculated with the spore suspension. The composition of the MI culture medium was as follows.

| | |
|---|---|
| glucose | 40 g |
| casein hydrolysate | 5 g |
| sodium nitrate | 3 g |
| potassium dihydrogen phosphate | 2 g |
| potassium chloride | 0.5 g |
| magnesium sulfate | 0.5 g |
| iron sulfate | 0.01 g |
| in 1000 ml of tap water | |

Before sterilization the pH value of the culture medium was adjusted to 6.0 and the sterilization was carried out at 121°C for 25 minutes. The culture was shaken on a shaking apparatus (250 rpm, 2.5 cm amplitude) for 3 days, then 100 ml of culture medium A2 sterilized in 50 Erlenmeyer flasks of 500 ml volume each at 121°C for 25 minutes were inoculated.

| Composition of the culture medium A2: | |
|---|---|
| glucose | 80 g |
| trypcasin | 10 g |
| in 1000 ml of tap water | |

The sterilization was performed at 121 °C for 25 minutes. The flask was shaken on a shaking apparatus (250 rpm, 2.5 cm amplitude) at 25 °C. The active substance content of the fermentation broth was determined by using HPLC method. The fermentation was continued for 168 hours and mycophenolic acid was isolated from the fermentation broth. From 5 L of fermentation broth containing 3100 mg/liter of mycophenolic acid, the product was isolated as follows.

After completion of the fermentation the pH value of the fermentation broth was adjusted to 3.2 ± 0.2 by adding sulfuric acid of 20 w%, then the acidic fermentation broth was stirred with 2500 ml of methylene chloride for one hour. Subsequently, after separating the phases the aqueous phase was extracted with 3x1250 ml of methylene chloride as described above. The combined methylene chloride extract containing mycophenolic acid was extracted with 1x500 ml and 2x250 ml of 5 w% sodium hydrogen carbonate solution. The combined aqueous alkaline solution containing mycophenolic acid sodium salt was cooled below 20 °C and the pH value of the solution was adjusted to 3.2 ± 0.2 by using 20 w% sulfuric acid solution. The acidic solution was extracted with 3x280 ml of methylene chloride, then the combined methylene chloride extract was evaporated under reduced pressure. The evaporation residue was dissolved in 88 ml of isopropyl alcohol at 80°C and clarified with 1.1 g of active carbon for 30 minutes. After filtering the active carbon the filter surface was washed twice with hot isopropyl alcohol. The combined isopropyl alcohol filtrate was evaporated under reduced pressure, then the crude product was dissolved in 88 ml of isopropyl alcohol at 80°C. Subsequently, the solution obtained was cooled to room temperature and allowed to stand at 0 to 5 °C overnight. The crystals were filtered and washed once with cooled isopropyl alcohol and twice with n-hexane. The product obtained was dried at 50 to 60 °C under reduced pressure. Subsequently, the dried product was dissolved in 55 ml of ethanol at an external temperature of 60 °C, then 22 ml of deionized water were dropwise added under stirring to the solution obtained. The solution was cooled to room temperature and allowed to stand at 0 to 5 °C overnight. After filtration the crystals were washed once with a 4:10 mixture of ethanol and water and twice with n-hexane and dried finally under reduced pressure at 50 to 60 °C to give 9.0 g of pure mycophenolic acid.

Melting point: 141 °C.

The spectroscopical data of the product are as follows:

Ultraviolet spectrum (in a concentration of 20 µg/ml in 96 % ethanol with a layer thickness of 1 cm):

| λₘₐₓ(nm) | ε | log ε | Assignment |
|---|---|---|---|
| 215 | 44,640 | 4.650 | Aromatic band |
| 249 | 9250 | 3.966 | Conjugation band |
| 304 | 4950 | 3.694 | C=O n→π^{*} transition |

IR: 3415; 1745; 1710; 1625 cm⁻¹;
¹H-NMR (CDCl₃, δ _{TMS} = 0 ppm): 1,80 (s, 3H, 4-CH₃); 2,13 (s, 3H, 7'-CH₃); 2,30 (t, *J*=8 Hz, 2H, H₂-3); 2,42 (t, *J*=8 Hz, 2H, H₂-2); 3,37 (d, *J*=6,6 Hz, 2H, H₂-6); 3,76 (s, 3H, 6'-OCH₃); 5,16 (s, 2H, H₂-1'); 5,25 (t, *J*=6,6 Hz, 1H, H-5);
¹³C-NMR (CDCl₃, δ_{TMS} = 0 ppm): 11,5, q (7'-CH₃); 16,0, q (4-CH₃); 22,5, t (C-6); 32,7, t (C-2); 34,1, t (C-3); 60,9, q (6'-OCH₃); 70,0, t (C-1'); 106,3, s (C-3a'); 116,7, s (C-7'); 122,0, s (C-5'); 122,9, d (C-5); 133,8, s (C-4); 144,0, s (C-7a'); 153,6, s (C-4'); 163,6, s (C-6'); 172,9, s (C-3'); 179,4, s (C-1).
Mass spectra
Electron ionization (EI) mass spectrum
Characteristic spectral data:
EI (70 eV): m/z 320 ([M]^{+•}; m/z 302 ([M-H₂O]^{+•}); m/z 247 ([M-^{•}CH₂CH₂-COOH]⁺); m/z 229 ([247-H₂O]⁺); m/z 207 ([C₁₁H₁₁O₄]⁺); m/z 159 ([C₁₀H₇O₂]⁺).
Chemical ionization (CI) mass spectrum
Characteristic spectral data: CI (i-butane): m/z 321 ([M+H]⁺); m/z 320 ([M]^{+•}); m/z 303 ([M+H - H₂O]⁺).

### Example 3

5 L of productive culture medium A2 sterilized at 121 °C for 45 minutes in a laboratory fermentor of 5 L working volume were inoculated with 250 ml of the shaken culture inoculum prepared as described in Example 2. After inoculation the fermentor was operated at 25 °C by bubbling 120 L/h of sterile air and the stirrer at a rate of 400 rpm. The fermentation was continued for 168 hours, then mycophenolic acid was isolated from the fermentation broth. The obtained fermentation broth of 4.9 L contained 2000 mg/l of mycophenolic acid at the end of fermentation. The mycophenolic acid was isolated from the fermentation broth as described in Example 2.

### Example 4

5 L of production medium A3 sterilezed in a laboratory fermentor at 121°C for 35 minutes were inoculated with 250 ml seed culture prepared as described in Example 2.

| Composition of the medium A3: | |
|---|---|
| glucose | 80 g |
| NZ Amine YTT (Quest) | 10 g |
| in 1000 ml of tap water | |

The fermentor was operated at 27°C with 120 L/h aeration and 400 rpm agitation. The fermentation was continued for 168 hours. The obtained 5 L fermentation broth contained 2550 mg/l of mycophenolic acid. The mycophenolic acid was isolated from the fermentation broth according to the Example 2.

### Example 5

A cell suspension was prepared from a culture of Streptomyces *griseoruber* strain No. 1/6 deposited as *Streptomyces* sp. No. 1/6 [NCAIM (P)B 001275] grown on CM agar slant.

| Composition of the agar culture medium CM: | |
|---|---|
| glucose | 25 g |
| peptone | 2 g |
| yeast extract | 1 g |
| magnesium sulfate - water (1:7) | 0.5 g |
| potassium dihydrogen phosphate | 5 g |
| agar in 1000 ml of distilled water | 20 g |

5 ml of the suspension were inoculated to 100 ml of sterilized inoculum culture medium MB in an Erlenmeyer flask of 500 ml volume.

| Composition of the culture medium MB: | |
|---|---|
| glucose | 20 g |
| malt extract | 30 g |
| yeast extract | 10 g |
| magnesium sulfate - water (1:7) | 1 g |
| in 1000 ml of tap water | |

Before sterilization the pH value of the culture medium was adjusted to 7.0 and the sterilization was carried out at 121 °C for 25 minutes. The cultures were grown on a shaking apparatus (250 rpm, 2.5 cm amplitude) at 28 °C for 3 days, then 5 ml each of the seed culture obtained were inoculated in 50 Erlenmeyer flasks of 500 ml volume each to 100-100 ml of sterilized bioconversion culture medium MBT each having the following composition:

| | |
|---|---|
| glucose | 20 g |
| malt extract | 10 g |
| soybean flour | 5 g |
| peptone | 10 g |
| magnesium sulfate - water (1:7) | 1 g |
| potassium dihydrogen phosphate | 1 g |
| in 1000 ml of tap water | |

Before sterilization the pH value of the culture medium was adjusted to 7.0. The sterilization was carried out at 121 °C for 25 minutes. Glucose sterilized separately was added portionwise to the culture medium at inoculation. The flasks were shaken at 28 °C by using a shaking apparatus (250 rpm, 2.5 cm amplitude) for 3 days. To the 72-hour cultures mycophenolic acid dissolved in ethanol was added to a final concentration of 0.1 mg/ml. The fermentation was continued for further 7 days following the addition. At the end of the bioconversion 2.0 L of acetone and 4.0 L of ethyl acetate were added to the fermentation broth of 4.0 L. Then the mixture was stirred for one hour. Subsequently, the phases were separated and the aqueous phase was again extracted with 4.0 L of chloroform. The emulsified organic phases obtained in the course of the extraction were centrifuged. Then the separated organic phase was evaporated and then the crude product obtained (2.0 g) was chromatographed on a column prepared from 100 g of Kieselgel 60 (particle size 0.063 to 0.2 mm; Reanal, Budapest) adsorbent. The crude product was applied onto the column with a mixture of chloroform, methanol and 99.5 % acetic acid (8.5 : 1.5 : 0.01) and in the course of chromatography the above mixture was used as developing solvent. At the column chromatography, fractions of 10 ml each were collected and the fractions were analysed by TLC on a Kieselgel 60 F₂₅₄ DC alufoil (Merck), using the developing mixture described above (detection at room temperature with 1 % of FeCl₃ in ethanol reagent). The fractions mainly containing mycophenolic acid amide were combined, then 40 ml of deionized water were added to the 120 ml solution obtained. The pH value of the mixture was adjusted to 4.0-4.5 by adding 1N sodium hydroxide solution. After separation of the phases the aqueous phase was extracted with 40 ml of chloroform. The combined extracts were evaporated under reduced pressure and the product obtained (0.3 g) was again subjected to chromatography on a column prepared from 30 g of Kieselgel 60 (particle size: 0.063 to 0.02 mm; Reanal, Budapest) adsorbent. The chromatographical column was prepared by using a mixture of methylene chloride and ethyl acetate containing 10% of ethyl acetate. For the elution the ethyl acetate content of this mixture was increased by 10 % in each subsequent elution. In the course of chromatography, fractions of 15 to 20 ml were taken and investigated by TLC under the conditions described above. (In the course of investigation a mixture consisting of benzene and ethyl acetate (1:2) was applied as developing system.) The product was dissolved from the column by using a mixture of methylene chloride and ethyl acetate containing 40 % of ethyl acetate. The fractions containing the product were evaporated under reduced pressure to obtain 0.2 g of chromatographically pure mycophenolic acid amide.
IR: 3433; 1737; 1665; 1622 cm⁻¹;
¹H-NMR (CDCl₃, δ _{TMS} = 0 ppm): 1,80 (s, 3H, 4-CH₃); 2,14 (s, 3H, 7'-CH₃); 2,31 (s, 4H, H₂-2 and H₂-3); 3,39 (d, *J*=6,3 Hz, 2H, H₂-6); 3,76 (s, 3H, 6'-OCH₃); 5,19 (s, 2H, H₂-1'); 5,21 (t, *J*=6,3 Hz, 1H, H-5);
¹³C-NMR (CDCl₃, δ_{TMS} = 0 ppm): 11,4, q (7'-CH₃); 16,0, q (4-CH₃); 22,5, t (C-6); 34,2, t (C-2); 34,9, t (C-3); 61,0, q (6'-OCH₃); 70,0, t (C-1'); 106,3, s (C-3a'); 116,7, s (C-7'); 122,0, s (C-5'); 123,0, d (C-5); 134,2, s (C-4); 144,1, s (C-7a'); 153,5, s (C-4'); 163,5, s (C-6'); 172,8, s (C-3'); 176,4, s (C-1).
Mass spectra
Electron ionization (EI) mass spectrum
Characteristic spectral data:
EI (70 eV): ([M]^{+•}) 319, C₁₇H₂₁NO₅; m/z 302, C₁₇H₁₈O₅ ([M - NH₃]^{+•}); m/z 301, C₁₇H₁₉NO₄, ([M - H₂O]^{+•}); m/z 261, C₁₅H₁₇O₄, ([M - ^{•}CH₂CONH₂]⁺ ); m/z 207, C₁₁H₁₁O₄, ([M-^{•}C₅H₈CONH₂]⁺).
Chemical ionization (CI) mass spectrum
Characteristic spectral data:
CI (i-butane): ([M+H]⁺) 320; ([M]^{+•}) 319; m/z 303 ([M+H - - NH₃]⁺); m/z 207 ([M - ^{•}C₅H₈CONH₂]⁺).

### Example 6

The preparation of the inoculum culture and bioconversion were carried out with the cell suspension prepared from the culture of *Streptomyces resistomycificus* strain No. 1/28 [NCAIM (P)B 001276] grown on CM agar slant as described in Example 5.

At the end of the bioconversion the fermentation broth of 4.5 L volume was extracted as described in Example 4. By evaporation of the extracts under reduced pressure 3.8 g of crude product were obtained, which was purified on a chromatographic column prepared from 114 g of Kieselgel 60 adsorbent (particle size: 0.063 to 0.2 mm; Reanal). In the course of purification by chromatography a mixture of chloroform, methanol and 99.5% acetic acid (8.5:1.5:0.01) was employed as developing system. During chromatography fractions of 15 to 20 ml each were taken and analyzed by TLC as described in Example 5. A mixture comprising chloroform, methanol and 99.5% acetic acid (8.5:1:5:0.1) was employed as developing system. The fractions containing mainly hydroxymethyl-mycophenolic acid amide were combined and the solution was processed before the evaporation as described in Example 5. The product (0.4 g) obtained after evaporation of the solution was subjected to a further chromatography on a column prepared from 40 g of Kieselgel 60 (particle size: 0.063 to 0.2 mm; Reanal) adsorbent. The chromatographic column was prepared in chloroform and during elution mixtures containing chloroform and methanol were employed, the methanol content of which was increased by 1 % in each elution. During the chromatography fractions of 10 ml each were taken and analysed by TLC under conditions described above. The product was dissolved from the column by using chloroform containing 5 % of methanol. The fractions containing the product were evaporated and the product obtained (0.3 g) was further purified by using methanol as solvent on a column containing 300 ml of Sephadex LH-20 gel (Pharmacia, Sweden). In the course of gel filtration chromatography, fractions of 10 ml each were taken analysed by TLC under the conditions described above. The fractions containing the pure product were combined and evaporated under reduced pressure to obtain 0.22 g of chromatographically homogeneous hydroxymethylmycophenolic acid amide.

Spectroscopic data of the product obtained:
IR: 3339; 1735; 1657; 1615 cm⁻¹;
¹H-NMR (MeOH-*d*₄, δ _{TMS} = 0 ppm): 1,80 (s, 3H, 4-CH₃); 2,25 (s, 4H, H₂-2 and H₂-3); 3,38 (d, *J*=6,5 Hz, 2H, H₂-6); 3,78 (s, 3H, 6'-OCH₃); 4,66 (s, 2H, 7'-CH₂-OH); 5,25 (t, *J*=6,5 Hz, 1H, H-5); 5,40 (s, 2H, H₂-1');
¹³C-NMR (MeOH-*d*₄, **δ**_{TMS} = 0 ppm): 16,2, q (4-CH₃); 23,4, t (C-6); 35,3, t and 36,5, t (C-2 and C-3); 57,8, t (7'-CH₂-OH); 62,9, q (6'-OCH₃); 71,5, t (C-1'); 108,2, s (C-3a'); 122,0, s and 123,6, s (C-5' and C-7'); 124,2, d (C-5); 135,3, s (C-4); 146,8, s (C-7a'); 156,1, s (C-4'); 164,1, s (C-6'); 173,7, s (C-3'); 178,7, s (C-1).
Mass spectra
Positive (+) and negative (-) FAB spectra
Characteristic spectral data:
+ and - FAB (xenon, 9 kV): [M+H]⁺ 336, m/z 318 [M+H-H₂O)⁺; [M--H]- 334.

## Claims

1. A microbiological process for the preparation of compounds of general formula (I) 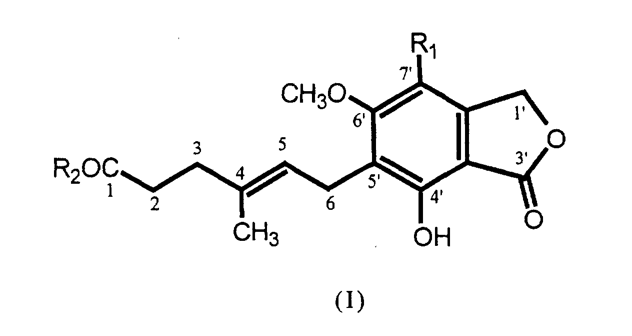 wherein R₁ means methyl or hydroxymethyl group and R₂ stands for hydroxyl or amino group, which comprises growing a *Penicillium waksmani* fungal strain able to biosynthesising mycophenolic acid on a culture medium containing assimilable carbon and nitrogen sources as well as mineral salts at 22 to 30 °C, separating the compound of general formula (I), wherein R, means methyl group and R₂ stands for hydroxyl group, from the fermentation broth and, if desired, purifying it and, if desired, bioconverting it
a) by using the cultures of *Streptomyces griseoruber* No. 1/6 actinomycete strain deposited at the National Collection of Agricultural and Industrial Microorganisms as *Streptomyces sp*. No. 1/6 under No. NCAIM (P)B 001275 to prepare a compound of general formula (I), wherein R₁ means methyl group and R₂ stands for amino group; or
b) by using the cultures of *Streptomyces resistomycificus* No. 1/28 actinomycete strain deposited at the National Collection of Agricultural and Industrial Microorganisms under No. NCAIM (P)B 001276 to prepare a compound of general formula (1), wherein R₁ means hydroxymethyl group and R₂ stands for amino group
on a culture medium containing assimilable carbon and nitrogen sources as well as mineral salts under submerged, acrated conditions up to completion of the bioconversion, then separating the compound of general formula (1) formed from the culture and, if desired, purifying it.

2. Process according to Claim 1, wherein the *Penicillium waksmani* strain deposited at the National Collection of Agricultural and Industrial Microorganisms under No. NCAIM (P)F 001269 or a mutant strain thereof, which is able to biosynthesising mycophenolic acid is cultivated.

## Patentansprüche

1. Mikrobiologisches Verfahren zur Herstellung von Verbindungen der allgemeinen Formel (I) 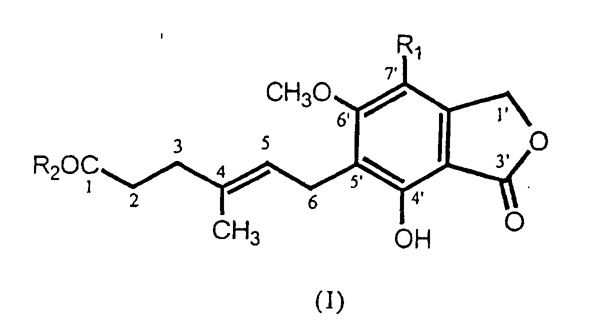 wobei R₁ eine Methyl- oder Hydroxymethylgruppe bedeutet und R₂ für eine Hydroxyl- oder Aminogruppe steht, wobei das Verfahren umfasst
Aufziehen eines Pilzstammes von Penicillium waksmani, der fähig ist, Mycophenolsäure zu biosynthetisieren, auf einem Kulturmedium, das Quellen assimilierbaren Kohlenstoffs und Stickstoffs wie auch Mineralsalze enthält, bei 22 bis 30°C,
Trennen der Verbindung der allgemeinen Formel (I), wobei R₁ eine Methylgruppe bedeutet und R₂ für eine Hydroxylgruppe steht, von der Fermentationsnährlösung und,
falls erwünscht, Reinigen dieser, und
falls erwünscht, Biokonvertieren dieser
a) durch Verwenden der Kulturen eines Actinomycete-Stammes von Streptomyces griseoruber Nr. 1/6, der bei der National Collection of Agricultural and Industrial Microorganisms als Streptomyces sp. Nr. 1/6 unter Nr. NCAIM (P)B 001275 hinterlegt ist, um eine Verbindung der allgemeinen Formel (I) herzustellen, wobei R₁ eine Methylgruppe bedeutet und R₂ für eine Aminogruppe steht, oder
b) durch Verwenden der Kulturen eines Actinomycete-Stammes von Streptomyces resistomycificus Nr. 1/28, der bei der National Collection of Agricultural and Industrial Microorganisms unter Nr. NCAIM (P)B 001276 hinterlegt ist, um eine Verbindung der allgemeinen Formel (I) herzustellen, wobei R₁ eine Hydroxymethylgruppe bedeutet und R₂ für eine Aminogruppe steht,
auf einem Kulturmedium, das Quellen assimilierbaren Kohlenstoffs und Stickstoffs wie auch Mineralsalze enthält, unter submersen, belüfteten Bedingungen bis zum Abschluss der Biokonversion,
dann Trennen der Verbindung der allgemeinen Formel (I), die von der Kultur gebildet wurde und, falls erwünscht, Reinigen dieser.

2. Verfahren nach Anspruch 1, bei dem der Stamm Penicillium waksmani, der bei der National Collection of Agricultural and Industrial Microorganisms unter Nr. NCAIM (P)F 001269 hinterlegt ist, oder ein zugehöriger Mutantenstamm, der fähig ist, Mycophenolsäure zu biosynthetisieren, kultiviert wird.

## Revendications

1. Procédé microbiologique pour la préparation de composés de formule générale (I) ; 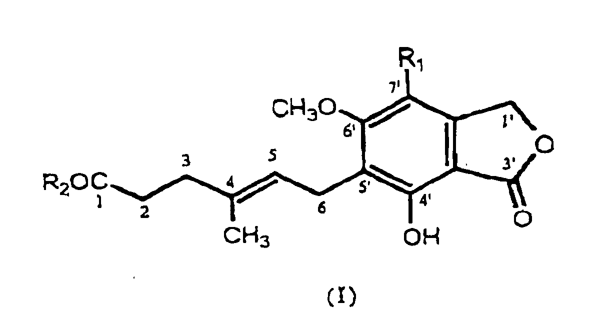 dans laquelle R₁ désigne un groupe méthyle ou hydroxyméthyle et R₂ désigne un groupe hydroxyle ou amino, qui comprend la croissance d'une souche fongique *Penicillium waksmani* capable de biosynthétiser l'acide mycophénolique sur un milieu de culture contenant des sources carbonées et azotées assimilables ainsi que des acides minéraux de 22 à 30°C, la séparation du composé de formule générale (I), dans laquelle R₁ désigne un groupe méthyle et R₂ désigne un groupe hydroxyle, à partir du bouillon de fermentation et, si souhaité, sa purification et, si souhaité, sa bioconversion
a) en utilisant les cultures de la souche d'actinomycète *Streptomyces griseoruber* n° 1/6 déposée auprès de la National Collection of Agricultural and Industrial Microorganisms en tant que *Streptomyces sp.* n° 1/6 sous le numéro de la NCAIM (P)B 001275 pour préparer un composé de formule générale (I), dans laquelle R₁ désigne un groupe méthyle et R₂ désigne un groupe amino ; ou
b) en utilisant les cultures de la souche d'actinomycète *Streptomyces resistomycificus n°* 1/28 déposée auprès de la National Collection of Agricultural and Industrial Microorganisms sous le numéro de la NCAIM (P)B 001276 pour préparer un composé de formule générale (I), dans laquelle R₁ désigne un groupe hydroxyméthyle et R₂ désigne un groupe amino
sur un milieu de culture contenant des sources carbonées et azotées assimilables ainsi que des sels minéraux dans des conditions d'immersion et d'aération jusqu'à la fin de la bioconversion, puis la séparation du composé de formule générale (I) formé à partir de la culture et, si souhaité, sa purification.

2. Procédé selon la revendication 1, dans lequel on cultive la souche *Penicillium waksmani* déposée auprès de la National Collection of Agricultural and Industrial Microorganisms sous le numéro de la NCAIM (P)F 001269 ou une souche mutante de celle-ci, qui est capable de biosynthétiser l'acide mycophénolique.
